(19) Europäisches Patentamt / European Patent Office / Office européen des brevets

(11) **EP 3 806 648 B1**

(12) # EUROPEAN PATENT SPECIFICATION

(45) Date of publication and mention
of the grant of the patent:
**12.11.2025 Bulletin 2025/46**

(21) Application number: **19729572.8**

(22) Date of filing: **14.06.2019**

(51) International Patent Classification (IPC):
**A23J 1/12** *(2006.01)* **A23J 3/18** *(2006.01)*
**B01D 61/14** *(2006.01)*

(52) Cooperative Patent Classification (CPC):
**A23J 1/12; A23J 3/18; B01D 61/149;**
B01D 2315/16

(86) International application number:
**PCT/EP2019/065726**

(87) International publication number:
**WO 2019/238941 (19.12.2019 Gazette 2019/51)**

(54) **NON-VITAL WHEAT PROTEIN AND ITS PRODUCTION PROCESS**

NICHT-VITALES WEIZENPROTEIN UND VERFAHREN ZU SEINER HERSTELLUNG

PROTÉINE DE BLÉ NON VITALE ET SON PROCÉDÉ DE PRODUCTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.06.2018 EP 18305739**

(43) Date of publication of application:
**21.04.2021 Bulletin 2021/16**

(73) Proprietor: **ROQUETTE FRERES
62136 Lestrem (FR)**

(72) Inventors:
• **GEDVILAS, Mindaugas
1201 GENEVE (CH)**
• **MAZONIENE, Edita
37123 PANEVEZYS (LT)**
• **JUOTKIENE, Rasa
29120 ANYKSCIAI (LT)**
• **TVARIJONAVICIUS, Danas
15203 VILNIUS (LT)**

(74) Representative: **Plasseraud IP
104 Rue de Richelieu
CS92104
75080 Paris Cedex 02 (FR)**

(56) References cited:
CN-A- 101 427 728    CN-A- 106 615 598
JP-A- H08 217 795

• SANA GAMMOH ET AL: "The effects of protein-phenolic interactions in wheat protein fractions on allergenicity, antioxidant activity and the inhibitory activity of angiotensin I-converting enzyme (ACE)", FOOD BIOSCIENCE, vol. 24, 19 May 2018 (2018-05-19), NL, pages 50 - 55, XP055497691, ISSN: 2212-4292, DOI: 10.1016/ j.fbio.2018.05.008
• MOSSES OKOT-KOTBER ET AL: "Activity and Inhibition of Polyphenol Oxidase in Extracts of Bran and Other Milling Fractions from a Variety of Wheat Cultivars", CEREAL CHEMISTRY, vol. 78, no. 5, 1 September 2001 (2001-09-01), US, pages 514 - 520, XP055497952, ISSN: 0009-0352, DOI: 10.1094/CCHEM.2001.78.5.514
• HASSAN HASSAN ET AL: "Corresponding Author: Nutritional and Functional Properties of Defatted Wheat Protein Isolates", AUSTRALIAN JOURNAL OF BASIC AND APPLIED SCIENCES, 1 January 2010 (2010-01-01), pages 348 - 358, XP055497772, Retrieved from the Internet <URL:https://pdfs.semanticscholar.org/4439/ 40d9814276444b1d074e4cd5506ec39bb12b.pdf> [retrieved on 20180807]

**(Cont. next page)**

EP 3 806 648 B1

- **WU Y V: "PROTEIN ISOLATE FROM AN EXPERIMENTAL HIGH-PROTEIN WHEAT AND FLOUR", JOURNAL OF AGRICULTURAL AND FOOD CHEMISTRY, AMERICAN CHEMICAL SOCIETY, BOOKS AND JOURNALS DIVISION, US, vol. 41, no. 7, 1 July 1993 (1993-07-01), pages 1048 - 1052, XP000381857, ISSN: 0021-8561, DOI: 10.1021/JF00031A006**
- **GEORGE LOOKHART ET AL: "ANALYTICAL TECHNIQUES AND INSTRUMENTATION Separation and Characterization of Wheat Protein Fractions by High-Performance Capillary Electrophoresis 1", 1 January 1995 (1995-01-01), XP055497949, Retrieved from the Internet <URL:http://www.aaccnet.org/publications/cc/backissues/1995/documents/72_527.pdf> [retrieved on 20180807]**
- **FEILLET P: "Wheat albumins and globulins. (translated)", ANNALES DE TECHNOLOGIE AGRICOLE, INSTITUT NATIONAL DE LA RECHERCHE AGRONOMIQUE <PARIS>, FR, vol. 25, no. 2, 1 January 1976 (1976-01-01), pages 203 - 216, XP009507303, ISSN: 0003-4223**

**Description**

## FIELD OF THE DISCLOSURE

This disclosure relates to a non-vital wheat protein, preferably wheat albumin, which is characterized by its high emulsion activity, high foam capacity and its low bitterness. This disclosure also relates to a process that allows industrial production of such non-vital wheat protein, preferably wheat albumin. Finally, this disclosure relates to industrial uses, including food, feed, cosmetic and pharmaceutical applications.

## BACKGROUND OF THE DISCLOSURE

Wheat is well known as a seed that allows to industrially produce starches, lipids and proteins which will be used in food, feed, cosmetics and pharmaceutical industries. The main components seeked are wheat starch and wheat gluten. The two methods most commonly used in the production of starch and gluten from wheat are the so-called Halle or fermentation process and the Martin's process.

In the fermentation process, the mashed grain is allowed to undergo a prolonged process of spontaneous fermentation, whereby the properties of wheat gluten are so modified that they no longer seriously interfere with the washing-out of the starch. The fermented mash is then agitated with water in a finely perforated vessel, thus causing a suspension of starch in water which escapes through the perforated walls of the vessel. Putrefactive processes, which occur during the fermentation, render the method extremely offensive in operation. Moreover, the process does not allow the wheat gluten to be recovered in a commercial form.

In the Martin's process, wheat flour is worked to a dough with water, and the dough stirred and kneaded by grooved rollers which repeatedly roll out and turn over the dough on a fixed bed flanked by sieve surfaces, while the starch is washed out by jets of water. The operation of this process is extremely tedious and has the added disadvantage of yielding a relatively large proportion of inferior starch strongly contaminated with finely divided gluten from which it cannot be separated by ordinary methods of purification.

In all wheat starch extraction processes, proteins are separated in two main streams: wheat gluten and wheat albumins. Wheat gluten is separated at the beginning as it is insoluble. Wheat albumins are concentrated in the soluble fraction. All processes will lead to a liquid fraction which is called wheat solubles. Such typical byproducts of wheat milling refinery typically contains 30% of residual starch, 4-7% ash, and 15-35% soluble proteins. These proteins are mainly wheat albumins.

Wheat solubles are mainly used in the feed and fermentation industries. In the food field, some companies have succeeded to purify wheat albumins in order to sell it in the human food markets. For example, Nisshin Pharma discloses in JP2009000017686 a process in order to obtain a purified fraction of wheat albumin. This process comprises the aqueous extraction of whole wheat flour, precipitation of contaminating compounds and other less temperature stable proteins by boiling and ultrafiltration. This purified fraction can be consumed as such, or in a soup (see "Shelf Life of Drink and Powdered Soup Containing Wheat Albumin", from Miyazaki and al.). Hassan, in 2010, also explores valorization of wheat albumins. In its work, the conclusion is that wheat albumins are interesting for their functional properties. Unfortunately, foaming capacity (also called whippability) can only reach 1,68 ml of foam per ml of solution (or 168% of foam volume if expressed in percentage) and this value is reached at a very alkaline pH around 9- 10 which can be harmful to the proteins (see Table 10 of Hassan 2010). As more foaming protein are seeked by industry, wheat albumin functional properties need to be upgraded, allowing to express more foam.

Another main drawback of wheat albumin is that it has a very strong bitterness effect when consumed. Food industry is not a field of use because of this extreme bitterness. When food companies includes such wheat solubles containing albumins, they must add in their formulation compounds which aim to cover/mask the bitterness. US9259454 from Kao Corporation teaches for example a solid composition comprising wheat albumin and maltitol, sorbitol, lactitol, or any combination thereof, intending to deliver a food formulation based on wheat albumin and without bitter off-taste.

Lastly, wheat solubles have also a very low emulsification activity, which avoids some specific uses in food industry like calf-milk replacement or ice cream industry. If the man skilled in the art can get a rid of bitterness by adding polyols, he cannot achieve a high emulsifying albumin rich fraction.

Hassan et al., Australian Journal of Basic and Applied Sciences 2010, discloses the provision of non-vital wheat proteins, notably wheat albumin.

CN 106615598 A relates to an electron beam irradiation combined enzyme method applied to wheat germ protein.

CN 101427728 A relates to a method for improving the performance of wheat germ protein by using ultrasonic waves.

JP H08217795 A relates to a wheat protein hydrolysate having good solubility and transparency properties for applications in cosmetics.

Wu, Journal of agricultural and Food Chemistry 1993, is a scientific article in which wheat and wheat flour are used to prepare protein isolates.

However, none of these prior art documents discloses a process of preparation of a native wheat albumin having an emulsifying activity above 600 mL of oil per g of protein, notably wherein the albumin fraction is subjected either to a microfiltration, cationic chromatography or ultrafiltration.

In conclusion, there is still a need for a purified and enriched wheat albumin fraction which has a low bitterness off-flavor without adding external compounds and which possess high emulsification capacities and high foaming capacity (also called whippability).

## SUMMARY OF THE DISCLOSURE

The present invention is defined by the subject-matter of claims 1 and 2 hereafter.

An object of the disclosure is a non-vital wheat protein, preferably wheat albumin, more preferably native wheat albumin which is characterized by an emulsifying activity (see test A below) above 500g of oil per g of protein, preferably above 600, and more preferably above 1000.

The protein of the disclosure has a low bitterness off-flavor when consumed by a human panel. Such bitterness can also be evaluated by known methods of the art including electronic tongue.

The protein of the disclosure has also a whippability property (also called foaming capacity) above 250%, preferably above 300%

The protein of the disclosure of the embodiments above has a richness above 80% in proteins, expressed as N x 6.25 on dry matter, preferably above 90%.

The porcess according to the invention allows production of the protein of the disclosure above, which comprises the following steps:

1. starting with wheat seeds, extracting external fibers, wheat germ, starch and wheat gluten and obtaining a fraction called wheat solubles,
2. applying microfiltration to wheat solubles in order to obtain a microfiltration permeate,
3. applying cationic chromatography to microfiltration permeate allowing production of fractions of enriched wheat albumins,
4. applying ultrafiltration to fractions of enriched wheat albumin above, together or alone, in order to obtain a more albumin-enriched retentate,
5. concentrating and/or drying the albumin-enriched retentate above, characterized in that cationic chromatography of step 3 comprises the following steps :

   a. Microfiltration permeate is fed on a cationic resin, in order to bind proteins
   b. Different eluents are fed on the resin in order to release and separate proteins bound on the resin wherein the the different eluents of step b) are consecutively :

      a) 20mM malic acid and 3mM sodium carbonate solution at pH 6,2
      b) 20mM malic acid, 3mM sodium carbonate and 0,5 M NaCl solution at pH 6,2
      c) 30mM sodium carbonate and 0,45M NaCl solution at pH 12

## BRIEF DESCRIPTION OF THE FIGURES

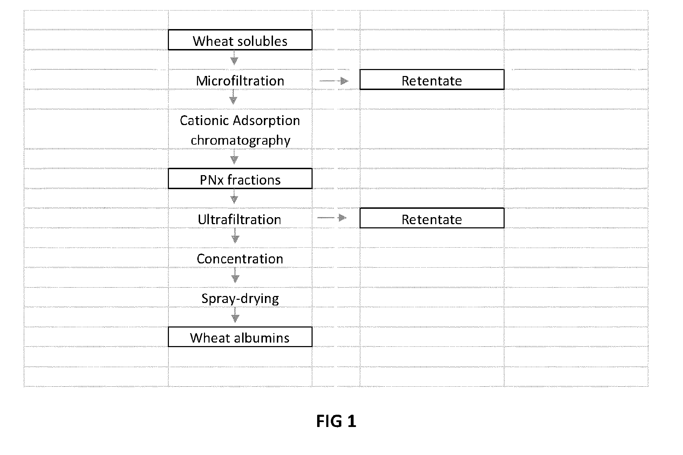

Figure. 1 represents the wheat solubles purification process of the disclosure
Figure 2 represents the chromatography peaks showing PN1, PN2 and PN3 peaks

## DETAILED DESCRIPTION OF THE DISCLOSURE

In this disclosure, "non-vital wheat protein" must be understood as a protein obtained from wheat seed excluding wheat gluten. Wheat gluten, composed of gliadins and glutenins, can be easily characterized by its viscoelastic properties. "Vital" property can be better understood by reading "Codex Standard for Wheat Protein Products Including Wheat Gluten (Codex Stan 163-1987, Rev. 1-2001)" which precises us that "Vital wheat gluten is characterized by its property of high viscoelasticity as hydrated. Devitalized wheat gluten is characterized by its reduced property of viscoelasticity as hydrated due to denaturation". By "non-vital wheat protein", man skilled in the art must understand all wheat proteins, including albumins but excluding all wheat gluten.

According to the present disclosure, "wheat" must be understood as a grass widely cultivated for its seed, a cereal grain which is a worldwide staple food. The many species of wheat together make up the genus *Triticum;* the most widely grown is common wheat (T. *aestivum*).

According to this disclosure, "protein" must be understood as large biomolecules, or macromolecules, consisting of one or more long chains of amino acid residues. Such protein can be native or modified, including hydrolysis or chemical modifications.

In this disclosure, "emulsion" must be understood as a mixture of two or more liquids that are normally immiscible (unmixable or unblendable). Emulsions are part of a more general class of two-phase systems of matter called colloids. Although the terms colloid and emulsion are sometimes used interchangeably, emulsion should be used when both phases, dispersed and continuous, are liquids. In an emulsion, one liquid (the dispersed phase) is dispersed in the other (the continuous phase). Examples of emulsions include vinaigrettes, homogenized milk, mayonnaise, and some cutting fluids for metal working.

In this disclosure, "emulsion activity" must be understood as the maximal quantity of oil which can be emulsified with water by one gram of protein.

In this disclosure, "Albumin" must be understood as a family of globular proteins. All the proteins of the albumin family are water-soluble, moderately soluble in concentrated salt solutions, and experience heat denaturation.

In this disclosure, "Bitterness" must be understood as the most sensitive of the tastes, and many perceive it as unpleasant, sharp, or unpleasant, but it is sometimes desirable and intentionally added via various bittering agents. Common bitter foods and beverages include coffee, unsweetened cocoa, South American mate, bitter gourd, olives, citrus peel, many plants in the family *Brassicaceae,* dandelion greens, wild chicory, and escarole. The ethanol in alcoholic beverages tastes bitter,[30] as do the additional bitter ingredients found in some alcoholic beverages including hops in beer and orange in bitters. Quinine is also known for its bitter taste and is found in tonic water

As described above, fan object of the disclosure is a non-vital wheat protein, preferably wheat albumin, more preferably native wheat albumin which is characterized by an emulsifying activity (see test A below) above 500g of oil per g of protein, preferably above 600, more preferably above 1000.

As exemplified below, wheat solubles produced by wheat refinery industry concentrates wheat albumin. By native wheat albumin, it is intended an albumin which exists in its natural state, which is not hydrolyzed. Albumins are well known for their properties including foaming, transparency and emulsifying power. Unfortunately, wheat solubles, even concentrated in albumin have a low emulsifying power or emulsifying activity. Industrial applications including food, feed, cosmetic and pharma cannot use these albumin source as functional compound in their products.

Emulsifying activity is defined above. The man skilled in the art is aware of various protocols which can help to measure this emulsifying activity. Emulsifying properties can be commonly described by two factors : emulsifying activity (EA) and emulsion stability (ES). (Boye & al. 2010a). Emulsifying activity (EA) can be defined by the maximum quantity of oil that can be dispersed in a water solution containing a known quantity of emulsifier before breaking or inversion of emulsion phase (Sherman, 1995).

On the basis of prior art, the Applicant of the present disclosure has developed the Test A described here below allowing to easily, quickly and surely quantify the emulsifying properties:

1. introduction of 0,2g of protein sample in 20ml of water,
2. homogenization with the help of Ultraturax IKA T25 during 30 sec at 9500 rpm,
3. addition of 20ml of corn oil under homogenization in same conditions as step 2 above,
4. centrifugation 5 minutes at 3100g.

   a. in case of good emulsion (no break or inversion in the emulsion), a new experiment is performed after raising quantity of water and oil of 50%.
   b. in case of bad emulsion (break or inversion in the emulsion), a new experiment is performed after lowering quantity of water and oil of 50%.

5. the maximum oil quantity (called Qmax, in ml) which can be emulsified is so determined iteratively.
6. 

$$\text{Emulsifying capacity} = (Qmax / 0,2)*100$$

Such emulsifying capacity analysis can be done at various pH, in order to evaluate its impact.

The protein of the disclosure above has a low bitterness off-flavor when it is consumed by a human panel. Such bitterness can also be evaluated by know methods of the art including electronic tongue.

Evaluation of bitterness can be easily and precisely done with the help of a trained human panel. Preferred protocol for such evaluation is described herebelow:

Six panelists trained to detect bitter taste are chosen. The tasting matrix is 5% suspension in water (Evian), followed by manual homogeneisation, and pasteurized 70°C/30min. Panel tasted each product in the same order and shared feelings about bitter taste experience. "Bitter" descriptor is particularly tracked.

Bitterness can also be evaluated with the help of an electronic tongue, e.g. Electronic Tongue SA402B which is manufactured and sold by Insent. A preferred protocol for such evaluation is explained below :

1. Weigh about 4 g of sample into a 250.0 mL volumetric flask,
2. Add 100 mL of purified water to the sample, and mix on a mechanical shaker for about 60 min,
3. Centrifugation of the suspension at about 3000 rpm for about 10 min,
4. Transfer of the supernatant to 2 sampling cups for analysis with the electronic tongue, which consists of introduction of the electronic tongue inside the supernatant and waiting for a stabilized measure (Bitteness but also Umami, Astrengency and Richness).

The protein of the disclosure has also a whippability property (also called foaming capacity) above 250%, preferably above 300%.

Whippability, also called foaming capacity, can be analyzed according to the following procedure, called TEST B in the present specification:

✔ Dispersion of 1g of sample with 40 ml of distilled water (Volume called below Vi),
✔ Stirring with Ultraturax IKA T25 during 30 sec at 8000 rpm,
✔ Second stirring with Ultraturax IKA T25 during 8 min at 9500 rpm,
✔ Transfer in a 100ml graduated test tube
✔ Measure volume of foam (Volume called below Vf), and liquid directly and after 90 min
✔

$$\underline{\text{Whippability}} = \text{Vf (Foam volume)} / \text{Vi (Volume before stirring).}$$

Whippability can also be expressed in percentage

$$\underline{\text{Whippability}} = ( \text{Vf (Foam volume)} / \text{Vi (Volume before stirring) } )* 100$$

The protein of the disclosure presenting the two embodiments above has a richness, in expressed $N \times 6.25$ on dry matter, above 80%, preferably above 90%.

Richness of albumin can be measured by well-known protocols known by the man skilled in the art, including electrophorese and nitrogen dosage. Best preferred protocol involves Kjeldahl analysis protocol.

The process according to the invention allows production of the protein according to the disclosure and, which comprises the following steps:

1. starting with wheat seeds, extracting external fibers, wheat germ, starch and wheat gluten, to obtain a fraction called wheat solubles
2. applying microfiltration to said fraction of wheat solubles, in order to obtain a microfiltration permeate
3. applying a cationic chromatography to said microfiltration permeate, allowing production of fractions of enriched wheat albumins,
4. applying an ultrafiltration step to said fractions of enriched wheat albumin, together or alone, in order to obtain a more albumin-enriched retentate,
5. concentrating and/or drying the albumin-enriched retentate above.

For step 1, well-known protocol can be applied in order to obtain wheat solubles.

As an example of a process suitable to recover wheat solubles, wheat grain is soaked with warm water and the ground grinded by a roller mill. During grinding, bran and germ are separated by an air flow and sieving, so that a wheat flour is obtained. Such flour is then mixed with 2 parts of water and stirred for 10 min in order for glutenin and gliadin to form a network, producing viscoelastic gluten. This flour pulp is then centrifuged. Starch, fibers and gluten paste is deposited. A remaining supernatant water, including salts, sugars and also wheat albumins is obtained, called "wheat solubles".

Wheat solubles, for example, are obtained from the separation stream of wheat "B" starches resulting from the separation of starch in the wet wheat starch milling process. B starch or "second starch" is starch consisting essentially of a preponderant proportion of small starch granules or of damaged granules. Besides this B starch, it is known that wheat solubles also contain non-negligible quantities of high-molecular-weight proteins capable of constituting a nitrogen source for microorganisms of interest.

For step 2, microfiltration removes residual high molecules and helps to get a filtered and clear wheat solubles in the permeate. Ceramic microfiltration membranes of 0,1 um pore size with a permeability gradient (GP) are preferred. Microfiltration (MF) permeate is led to the next step, while the retentate after washing out (diafiltration), rich in protein and oil, can be used for high protein feed production or recycled. Microfiltration is preferably done in a 3 consequential stack system, in which each next step is fed by the retentate of the former step, and the permeate is collected together from all 3 stacks. Diafiltration of the final retentate is not performed, but might be done in the 3rd or additional stack.

Step 3 consists in chromatographic separation of specific enriched albumins fractions. Preferably a cationic exchange chromatography is used. Macroporous beads of a hydrophilic copolymer of glycidyl methacrylate with propylsulfonic groups are prefered as a cation exchanger. The resin is pre-conditioned by low-pH (2,5) low-ionic strength acid (HCl or lactic, or malic) solution. Microfiltration permeate is adjusted to pH 3,8-4,0 by acid (HCl or lactic, or malic) and passed through the resin, until saturation of protein absorption (which can be detected when protein leaks at the output of resin). Unbound compounds containing mainly fibers in the so-called raffinate stream can be directed to a soluble fiber recovery or just recycled back to the original stream starch process water (possibly after the optional nano- or ultrafiltration). Bound protein is recovered from the resin with increasing pH & ionic strength gradient (NaOH and NaCl solutions, buffered by malic acid or carbonic acid are used) in 3 to 6 separate peaks, preferably 3 peaks.

The process of the invention is characterized in that cationic chromatography of step 3 comprises the following steps :

    a. Microfiltration permeate is fed on a cationic resin, in order to bind proteins
    b. Different eluents are fed on the resin in order to release and separate proteins bound on the resin

Furthermore, the process of the invention is characterized in that the different eluents of step b) are consecutively :

    i) 20mM malic acid and 3mM sodium carbonate solution at pH 6,2
    ii) 20mM malic acid, 3mM sodium carbonate and 0,5 M NaCl solution at pH 6,2
    iii) 30mM sodium carbonate and 0,45M NaCl solution at pH 12

For further processing, the peaks can be forwarded separately or mixed into so-called PN1, PN2 and PN3 fractions according notably to their palatability. The three separate protein fractions are being collected with the following characteristics:

- PN1: pH 6,1-6,3; conductivity 3-3,5 mS; low bitterness
- PN2: pH 6,1-6,4; conductivity 45-50 mS; low bitterness;
- PN3: pH 12,0-12,2; conductivity 45-50 mS; high bitterness

In step 4, protein fractions are concentrated by ultrafiltration (UF) with PES membranes in the tangential flow filtration (TFF) systems. Different cut-offs can be used from 1 to 6 kDa. To increase the protein content, at the end of ultrafiltration, PN2 fraction is diafiltered with reverse osmosis quality water. PN3 fraction before UF requires additional pH regulation (down to 5,5-6) and clarification by a centrifuge. UF permeate or diapermeate can be recycled (optionally after reverse osmosis filtration) to the starch production as PW. After this step the purified protein fraction of 9-10% dry matter can be obtained, even preferably 10-20%, even more preferabbly from 20 to 30%.

Step 5 aims to remove water. Possible solutions are ultrafiltration, short-residence-time evaporation or forward osmosis. In case of forward osmosis, osmotic agent is to be reconcentrated by reverse osmosis or evaporation. The filtrate or condensate is reused back into starch process.

Step 6 aims to dry protein. Spray-dryer is prefered. In general, product is non-sticky and easy to dry. Protein isolates are obtained as a light-weighed cream-colored dry powder with a dry matter content of around 95% and a protein content of 85 - 97%. Microagglomerated product might be desired for better commercial properties, which would require special drying solutions like spray/ fluid bed combination

The disclosure will be better understood with the following examples.

## **EXAMPLES**

Example 1 : Production of wheat solubles by a common process of the prior art

Wheat grain is tempered for 8h in water heated at 28°c. Water is removed and ground by a roller mill. After grinding, bran and germ are separated with combination of 1400, 530 and finally 300 microns sieving, in order to obtain a wheat flour with approx. 14% moisture and 0,8 % ash (dry basis). The flour obtained is mixed with 2 parts of water and stirred for 10 min so that glutenin and gliadin form a network, producing viscoelastic gluten. This flour pulp is then centrifuged at 3-10 x1000 G-force for 5 min. Starch, fibers and gluten paste are deposited. A remaining supernatant water, including salts, sugars and also wheat albumins, is obtained and called "wheat solubles". The average composition of such wheat solubles is summarized in Table 1 below :

**Table 1: Average composition of wheat solubles**

| Analysis | Units | Values |
|---|---|---|
| Dry matter | % | 5,5-7,5 |
| Total protein | % dry matter | 19-22 |
| Soluble protein | % of total protein | 47-51 |
| Soluble fibers | % dry matter | 14-17 |
| Saccharides | % dry matter | 33-35 |
| Lipids (crude fat) | % dry matter | 2-3 |
| Org. acids (lactic, acetic, etc.) | % dry matter | 15-25 |

Example 2: Production of bitterness-free and high emulsifying activity wheat albumin extracts

1000 kg of wheat solubles containing 20% of proteins on dry matter are involved in this example. Wheat solubles are fed in a membrane fractioning microfiltration unit, more precisely PALL ceramic microfiltration membranes of 0,1 um pore size with permeability gradient (GP). Microfiltration permeate will be fed to the next step, while the retentate after washing out (diafiltration) is discarded. A 3 consequential stack system is used, where each next step is fed by the retentate of the former step, and the permeate is collected together from all 3 stacks. Diafiltration of the final retentate is not performed, but might be done in the 3rd or additional stack. Filtration is ended after having reached a VCF (Volume Concentration Factor) of 5. Main parameters of microfiltration (MF) are summarized in table 2 below:

**Table 2: Main parameters of microfiltration**

| | |
|---|---|
| Temperature, °C | 45-60 |
| Inlet pressure, bar | 2,5 |
| Transmembrane pressure, bar | 1 |
| Recirculation rate, m3/h | 33 |
| Linear velocity of retentate, m/s | 5 |
| Average flux, l/h/m2 | 50 |

This step produces 722 kg of clarified permeate containing 6,5 kg of proteins. This permeate will be then purified by chromatography, more precisely involving a cationic exchange chromatography solid phase. Macroporous beads of a hydrophilic copolymer of glycidyl methacrylate with propylsulfonic groups are used as a cation exchanger. Beads are pre-conditioned by low-pH (2,5) low-ionic strength acid (HCl or lactic, or malic) solution and packed in a standard exchange column. Clarified permeate is adjusted to pH 3,8-4,0 with acid (HCl or lactic, or malic) and fed on the resin at 2 cm/min of linear velocity and 20°C. A first unbound stream, also called raffinate, representing 840kg at 2,3% dry matter is discarded. Bound protein is recovered from the resin with increasing pH & ionic strength step-wise gradient, allowing production of three distincts peaks. To achieve such separation in 3 distincts peaks, 3 eluents are fed consecutively on the resin as described in Table 3.

**Table 3: description of the three eluents fed consecutively on the resin.**

| Eluent | Used for elution of fraction | Reagents | pH |
|--------|------------------------------|----------|-----|
| 1 | **PN1** | 20mM malic acid + 3mM $Na_2CO_3$ + NaOH | 6,2-6,4 |
| 2 | **PN2** | 20mM malic acid + 2,5mM $Na_2CO_3$ + 0,5M NaCl + NaOH | 6,2-6,4 |
| 3 | **PN3** | 30mM $Na_2CO_3$, 0,45M NaCl + NaOH | 12 |

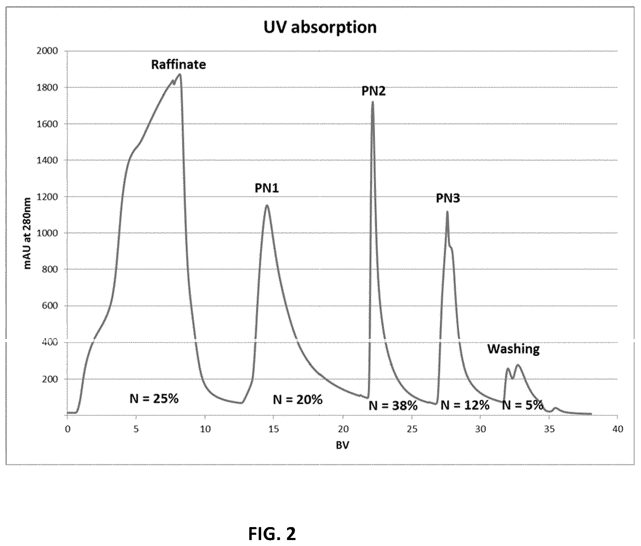

The three distinct peaks are detected using an UV detector, scanning at 280nm, placed at the output of the resin (see fig. 2). Peak protein fractions are collected with the following characteristics summarized in Table 4.

**Table 4: Peak protein fractions characteristics**

| Peak fractions | pH | Conductivity (mS) |
|----------------|-----|-------------------|
| PN1 | 6,1-6,3 | 3-3,5 |
| PN2 | 6,1-6,4 | 45-50 |
| PN3 | 12,0-12,2 | 45-50 |

This step produces 442 kg of PN1 fraction which contains 2,21 kg of proteins, 195 kg of PN2 fraction which contains 7,80 kg of proteins and 149 kg of PN3 fraction which contains 4,47 kg of proteins. PN1, PN2 and PN3 are then separately purified by ultrafiltration. PN3 fraction before ultrafiltration requires additional pH regulation (down to 5,5-6) and clarification by a centrifuge. PES membranes in tangential flow filtration (TFF) systems are preferred, with a cut-off of 6 kDa. In order to increase the protein content, at the end of ultrafiltration, PNx (PNx meaning PN1, PN2 and PN3 fractions) fractions are diafiltered with reverse-osmosis quality water. UF permeate or diapermeate can be recycled (optionally after RO filtration) to the starch production as PW. After this step PNx protein fractions reaching 9-10% DS and more than 90% richness in proteins, expressed in N x 6,25, can be obtained. Main ultrafiltration parameters are summarized in Table 5 below:

**Table 5: Main ultrafiltration parameters.**

| MWCO, kDa | 3-6 |
|-----------|-----|
| Temperature, °C | 6-10 or 55-60 |
| Inlet pressure, bar | 2 |
| Transmembrane pressure, bar | 1,5 |
| Recirculation rate, m3/h | Depends on the system |
| Linear velocity of retentate, m/s | 3 |
| Average flux, l/h/m2 | 3 or 10 (depending on temperature) |

All PNx protein fractions concentration are too low in dry matter to be spray-dried. Dry matter is concentrated to 20% by a ultrafiltration membrane filtration process, where permeate is discarded and retentate kept, concentrating wheat albumins. Main parameters of ultrafiltration (UF) are summarized in Table 6 below:

**Table 6: Main parameters of ultrafiltration**

| MWCO, kDa | 5 |
|-----------|-----|
| Temperature, °C | 6-10 or 55-60 |
| Inlet pressure, bar | 2 |
| Transmembrane pressure, bar | 1,5 |
| Recirculation rate, l/min | 3 |
| Linear velocity of retentate, m/s | 3 |
| Average flux,l/h/m2 | 3 or 14 (depending on temperature) |

Finally, all concentrated PNx protein fractions are dried in a Fujisaki spray-dryer. Protein isolates are obtained as a light-weighed cream-colored dry powder with a dry matter content of around 95% and a protein content of 85 - 97%. Parameters of currently used spray-dryer are summarized in the Table 7 below:

**Table 7: Parameters of spray-dryer**

| Temperature (in), °C | 190 |
| --- | --- |
| Temperature (out), °C | 70 |

Example 3: Comparison of disclosure and prior art

| | | Wheat solubles | PN1 | PN2 | PN3 |
| --- | --- | --- | --- | --- | --- |
| Emulsifying Activity at pH 4-5 | ml of oil/g of protein | 200,0 | 400,0 | 800,0 | 800 |
| Emulsifying Activity at pH 6-6,5 | ml of oil/g of protein | no data | 400,0 | 1000,0 | 800 |
| Emulsifying Activity at pH 8-8,5 | ml of oil/g of protein | 500,0 | 400,0 | 1000,0 | 800 |
| Whippability / Foam capacity (percentage) | % | 220 | 375,0 | 325,0 | 375,0 |
| Foam stability (90') | % | -100 | -92,0 | -46,0 | -91,0 |

**Table 8: Emulsifying activity at different pH, whippability/foam capacity and foam stability of wheat solubles, and PN1, 2 and 3 fractions.**

As described in table 8, PN2 and PN3 fractions can emulsify above 500 ml of oil per g of sample. Moreover only PN2 fraction allows to emulsify around 1000 ml of oil/g of protein.

In the other hand, it can be seen that, thanks to the process of the disclosure, PN2 and PN3 fractions present a whippability property above 300%, allowing production of a big quantity of foam with the same quantity of protein. Lastly, it can be seen that PN2 fraction allows production of very stable foam .

Example 4: Organoleptic comparison with a sensorial panel

Products :

- Pea isolate, NUTRALYS S85F batch W122K
- Hydrolyzed wheat gluten, NUTRALYS W batch E5166
- Whey protein isolate WPI 894
- Milk caseinate
- Microfiltration clarified permeate, feed of chromatography in the disclosure
- PN1 peak obtained in example 2
- PN2 peak obtained in example 2
- PN3 peak obtained in example 2

Six panelists trained to detect bitter taste have been involved in the test.

The tasting matrix is 5% suspension in water (Evian), after manual homogeneisation and pasteurization at 70°C/30min.

Panel tasted each product in the same order and shared feelings about bitter taste

The results are

- NUTRALYS S85F: strong pea/green smell and taste, **bitter,** astringent, chickpea broth
- Nutralys W : smell and taste are strong : green, rice, fermented (like Chinese green tea), **very bitter**
- WPI 894 : milk, cooked white egg, sandy, drying
- Milk caseinate : milk, washing water, sharp, sour and **bitter**
- Microfiltration clarified permeate, feed of chromatography in the disclosure : bitter
- PN2 and PN1 peak fractions : smell is quite neutral, a bit fruity, ice-tea like. Not bitter / astringent.
- PN3 fraction : very bitter, even bitter than the feed

In conclusion, Process of the disclosure can lead to a wheat protein isolate with no bitter taste (PN2), without formulating with polyols as taught in the prior art, but also to a very bitter wheat protein isolate (PN3). Very bitter fractions can be used in some industrial applications if needed.

Example 5: Antioxydant power of non-vital wheat protein of the disclosure

This example aims at evaluating antioxidant power of PN2 sample from example 4 versus common protein samples by the LUCS technology on the HaCaT human keratinocyte cell model.

LUCS approach is based on the production of cellular radical species following the addition in the culture medium of a photo-inducible fluorescent nucleic acid biosensor. The effect of light application in presence of the cellular biosensor triggers the production of singlet oxygen which in turn causes the production of ROSs (Reactive Oxygen Species) in a biochemical cascade linked to an increase of emitted fluorescence.

LUCS assay measures the ability of an antioxidant compound to scavenge ROSs in live cells and to neutralize oxidative stress. The effect is measured by a delay in the kinetic evolution of fluorescence emission. The approach has been standardized on high throughput 96-well plates to allow for reliable statistic analysis (EP2235505,US20110008783 and Derick S. et al. Sci Rep. 2017, 7(1):18069).

Prior to experiment, protein samples were digested following an *in vitro* gastrointestinal (GI) protocol. First, an amount of 5 g PN2 sample from example 4 was dissolved in 95 mL ultrapure water and 250 µL 0.3 M $CaCl_2$ under magnetic stirring for 1 hour at 37°C. Then, a gastric fluid made of 80 mL ultrapure water, 15 mL HCl 1 M and 50 µL $CaCl_2$ 0.3 M was added to the protein solution. Prior to pepsin addition, pH was adjusted to 2 with HCl 4 M. An amount of 0.5 g pepsin (from porcine gastric mucosa EC 3.4.23.1, CAS 9001-75-6, reference Sigma P7000) was added to the solution kept under magnetic stirring for 2 hours at 37°C. Every 30 min pH was measured and adjusted to 2 if required. Then, an intestinal fluid made of 175 mL phosphate buffered saline (PBS) solution pH 7.4, 35 mL ultrapure water, 400 µL CaCl2 0.3 M, and 4.5 mL NaOH 1 M was added to the GI system and pH was adjusted to 6.8 with NaOH 4 M if required. An amount of 1 g pancreatin (from porcine pancreas EC 232-468-9, CAS 8049-47-6, reference Sigma P7545) was added to the solution and the whole system was kept under magnetic stirring at 37 °C for 2h. Every 30 min pH was measured and adjusted to 6.8-7 if required. Once the *in vitro* GI digestion was completed, the solution was heated up at 90°C for 10 min for enzyme deactivation. Samples were centrifuged at 10 000 g for 10 min and supernatants were stored at -20°C.

A volume of 10 ml of DMEM (Dulbecco's Modified Eagle Medium) cell culture medium without FCS (Fetal Calf Serum) were added in 500 mg of each sample (whey protein, white egg protein and PN2 sample from example 4. Solutions (50 mg/ml w/v) were vortexed, followed by a centrifugation (8700 rpm, 10 min). Supernatants were aliquoted and kept at -20°C until the day of the experiment. The pH value of samples was 8 compatible with AOP assays. Prior to experiment, samples are then warmed up to 90°C for 10 min to inactivate digestive enzymes. The study was performed on human HaCaT cells. Cells were seeded in 96-well plates at a density of 75 000 cells/well in DMEM medium supplemented with Fetal Calf Serum (FCS) and kept in the incubator for 24h at 37°C/5% $CO_2$. Cells were then incubated in the presence of samples (8 concentrations obtained by serial log2 dilutions) during 4 h at 37°C/5% $CO_2$. Experiments were performed in DMEM medium without FCS. Two independent experiments were realized each on triplicate wells.

Cells were treated after the 4 h incubation with the fluorescent biosensor during 1 h and fluorescence was measured (RFU at 535 nm) according to a recurrent 480 nm LED application procedure (20 iterations) of the whole 96-well plate. Kinetic profiles were recorded.

Antioxidant cell index (AOP index) is calculated from normalized kinetic profiles according to the formula:

$$\text{AOP index (\%)} = 100 - 100 \left( {}_0\!\int^{20} \text{RFU sample} / {}_0\!\int^{20} \text{RFU control} \right)$$

Dose-response curves, obtained by compiling AOP indices according to logarithm (10) of the sample concentration, are submitted to a sigmoid fit according to the formula:

$$\text{AOP index} = \text{AOP index min} + (\text{AOP index max} - \text{AOP index min})/(1 + 10(\text{Log}(EC50-SC)*HS))$$

where SC = sample concentration and HS = Hill slope. EC50 (50% efficacy concentration), EC10 and EC90 are then evaluated when possible.

Table 9 below presents results:

**Table 9: Comparison of different samples EC50**

| Sample | EC50 (mg.mL$^{-1}$ dry matter) |
| --- | --- |
| PN2 sample from example 4 | 2,05 |
| Whey protein isolate (Prodiet F90 Instant, from INGREDIA) | Not detectable |
| White egg albumin (from LIOT) | Not detectable |

Results clearly show that the PN2 sample proteins possess a strong antioxidant activity versus common animal prior art proteins.

**Claims**

1. Process susceptible to produce native wheat albumin, having an emulsifying activity above 600 mL of oil per g of protein, said emulsifying activity being quantified according to test A, **characterized in that** it comprises the following steps:

    1. starting with wheat seeds, extracting external fibers, wheat germ, starch and wheat gluten and obtaining a fraction called wheat solubles,
    2. applying microfiltration to wheat solubles in order to obtain a microfiltration permeate,
    3. applying cationic chromatography to microfiltration permeate allowing production of 3 fractions of enriched wheat albumins,
    4. applying ultrafiltration to fractions of enriched wheat albumin above, together or alone, in order to obtain a more albumin-enriched retentate,
    5. concentrating and/or drying the albumin-enriched retentate above;
    **characterized in that** cationic chromatography of step 3 comprises the following steps:

        a. Microfiltration permeate is fed on a cationic resin, in order to bind proteins,
        b. Different eluents are fed on the resin in order to release and separate proteins bound on the resin;

    wherein the different eluents of step b)are consecutively:

        i) 20mM malic acid and 3mM sodium carbonate solution at pH 6.2
        ii) 20mM malic acid, 3mM sodium carbonate and 0.5 M NaCl solution at pH 6.2
        iii) 30mM sodium carbonate and 0.45M NaCl solution at pH 12.

2. Process according to claim 1, wherein the concentration step is carried out with an ultrafiltration.

**Patentansprüche**

1. Verfahren zur Herstellung von nativem Weizenalbumin mit einer emulgierenden Aktivität von über 600 mL Öl pro g Protein, wobei die emulgierende Aktivität gemäß Test A quantifiziert wird, **dadurch gekennzeichnet, dass** es die folgenden Schritte umfasst:

    1. Ausgehend von Weizensamen, Extrahieren von externen Fasern, Weizenkeimen, Stärke und Weizengluten und Erhalten einer Fraktion, welche als lösliche Weizenstoffe bezeichnet wird,
    2. Anwenden von Mikrofiltration auf die löslichen Weizenstoffe, um ein Mikrofiltrationspermeat zu erhalten,
    3. Anwenden von kationischer Chromatographie auf das Mikrofiltrationspermeat, welches die Herstellung von 3 Fraktionen angereicherter Weizenalbumine erlaubt,

4. Anwenden von Ultrafiltration auf obige Fraktionen angereicherten Weizenalbumins, zusammen oder einzeln, um ein stärker Albumin-angereichertes Retentat zu erhalten,

5. Konzentrieren und/oder Trocknen des obigen Albumin-angereicherten Retentats;

**dadurch gekennzeichnet, dass** die kationische Chromatographie von Schritt 3 die folgenden Schritte umfasst:

a. Das Mikrofiltrationspermeat wird einem kationischen Harz zugeführt, um Proteine zu binden,

b. Verschiedene Eluenten werden dem Harz zugeführt, um die an das Harz gebundenen Proteine freizusetzen und zu trennen;

wobei die verschiedenen Eluenten von Schritt b) nacheinander sind:

i) 20 mM Apfelsäure und 3 mM Natriumcarbonatlösung bei pH 6,2

ii) 20 mM Apfelsäure, 3 mM Natriumcarbonat und 0,5 M NaCl-Lösung bei pH 6,2

iii) 30 mM Natriumcarbonat und 0,45 M NaCl-Lösung bei pH 12.

2. Verfahren nach Anspruch 1, wobei der Konzentrationsschritt mit einer Ultrafiltration durchgeführt wird.

**Revendications**

1. Procédé susceptible de produire de l'albumine de blé native, ayant une activité émulsifiante supérieure à 600 mL d'huile par g de protéine, ladite activité émulsifiante étant quantifiée selon l'essai A, **caractérisé en ce qu'**il comprend les étapes suivantes :

1. en commençant avec des graines de blé, l'extraction de fibres externes, germe de blé, amidon et gluten de blé et l'obtention d'une fraction appelée solubles de blé,

2. l'application d'une micro-filtration à des solubles de blé afin d'obtenir un perméat de micro-filtration,

3. l'application d'une chromatographie cationique au perméat de micro-filtration permettant la production de 3 fractions d'albumines de blé enrichies,

4. l'application d'une ultrafiltration à des fractions d'albumine de blé enrichie ci-dessus, ensemble ou seules, afin d'obtenir un rétentat davantage enrichi en albumine,

5. la concentration et/ou le séchage du rétentat enrichi en albumine ci-dessus ;

**caractérisé en ce que** la chromatographie cationique de l'étape 3 comprend les étapes suivantes :

a. Le perméat de micro-filtration est acheminé sur une résine cationique, afin de lier des protéines,

b. Différents éluants sont acheminés sur la résine afin de libérer et séparer des protéines liées sur la résine ;

dans lequel les différents éluants de l'étape b) sont consécutivement :

i) une solution d'acide malique 20 mM et de carbonate de sodium 3 mM à pH 6,2

ii) une solution d'acide malique 20 mM, de carbonate de sodium 3 mM et de NaCl 0,5 M à pH 6,2

iii) une solution de carbonate de sodium 30 mM et de NaCl 0,45 M à pH 12.

2. Procédé selon la revendication 1, dans lequel l'étape de concentration est réalisée avec une ultrafiltration.

```
Wheat solubles
      │
      ▼
Microfiltration  ───►   Retentate
      │
      ▼
Cationic Adsorption
  chromatography
      │
      ▼
  PNx fractions
      │
      ▼
Ultrafiltration  ───►   Retentate
      │
      ▼
 Concentration
      │
      ▼
  Spray-drying
      │
      ▼
Wheat albumins
```

**FIG 1**

UV absorption

Raffinate

PN1

PN2

PN3

Washing

N = 25%  N = 20%  N = 38%  N = 12%  N = 5%

mAU at 280nm

2000
1800
1600
1400
1200
1000
800
600
400
200

0   5   10   15   20   25   30   35   40

BV

FIG. 2

15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009000017686 B
- US 9259454 B
- CN 106615598 A
- CN 101427728 A
- JP H08217795 A
- EP 2235505 A
- US 20110008783 A

**Non-patent literature cited in the description**

- **MIYAZAKI**. *Shelf Life of Drink and Powdered Soup Containing Wheat Albumin*
- **HASSAN et al.** *Australian Journal of Basic and Applied Sciences*, 2010
- **WU**. *Journal of agricultural and Food Chemistry*, 1993
- Vital'' property can be better understood by reading. *Codex Standard for Wheat Protein Products Including Wheat Gluten (Codex Stan 163-1987, Rev. 1-2001)*
- **DERICK S. et al.** *Sci Rep*, 2017, vol. 7 (1), 18069